(19) Europäisches Patentamt
European Patent Office
Office européen des brevets

(11) **EP 4 706 685 A1**

(12) **EUROPEAN PATENT APPLICATION**
published in accordance with Art. 153(4) EPC


(43) Date of publication:
**11.03.2026 Bulletin 2026/11**

(21) Application number: **24800230.5**

(22) Date of filing: **02.05.2024**

(51) International Patent Classification (IPC):
***A61K 47/69*** (2017.01) ***A61K 31/575*** (2006.01)

(52) Cooperative Patent Classification (CPC):
**A61K 31/575; A61K 47/69**

(86) International application number:
**PCT/KR2024/005931**

(87) International publication number:
**WO 2024/228549 (07.11.2024 Gazette 2024/45)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **02.05.2023 KR 20230057301**



(71) Applicant: **CARBOEXPERT Inc.**
**Daejeon 34134 (KR)**

(72) Inventors:
- **PARK, Jong Tae**
  **Daejeon 34094 (KR)**
- **KIM, Jae Han**
  **Daejeon 34071 (KR)**
- **KIM, Dae Ryeol**
  **Jeungpyeong-gun, Chungcheongbuk-do 27927 (KR)**
- **BAN, So Young**
  **Daejeon 34194 (KR)**
- **KWAK, Ji Yun**
  **Sejong 30153 (KR)**
- **LEE, Nan Young**
  **Daejeon 34185 (KR)**

(74) Representative: **WSL Patentanwälte Partnerschaft mbB**
**Kaiser-Friedrich-Ring 98**
**65185 Wiesbaden (DE)**


(54) **PHARMACEUTICAL FORMULATION COMPRISING LANOSTEROL, DERIVATIVE THEREOF, AND CYCLODEXTRIN**


(57) The present disclosure relates to a pharmaceutical formulation including lanosterol, a derivative thereof, and cyclodextrin. The pharmaceutical formulation, according to an aspect, exhibits enhanced water solubility and absorption in the body, and thus has the effect of increasing bioavailability.


FIG. 6A

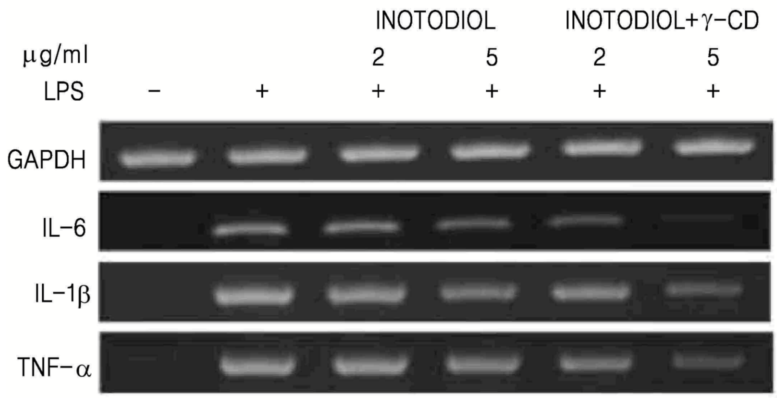


EP 4 706 685 A1

## Description

### Technical Field

The present disclosure relates to a pharmaceutical formulation including lanosterol and a derivative thereof and cyclodextrin.

### Background Art

Cyclodextrins have pharmaceutical applications and are used for solubilization or stabilization of many compounds, but these applications have limitations when applied to therapeutic agents. Cyclodextrins have may not form complexes with many compounds and are unsuitable for pharmaceutical applications [see J. Szejtli, Pharmaceutical Technology, 1991, 24-38; and US Patent No. 5,362,860]. In particular, the bioavailability of cyclodextrin mixtures is often unpredictable, and in fact, formation of a [drug:cyclodextrin] complex can often result in reduced bioavailability of the drug [see T. Loftsson, Pharmaceutical Technology, 1999, 12, 40-50; and Uekama, K, et al., CRC Critical Reviews in therapeutic Drug Carrier Systems, 1987, 3(1), 1-40].

Meanwhile, studies have been conducted to isolate useful compounds from Chaga mushrooms, and various compounds such as inotodiol, trametenolic acid, lanosterol, and inonotsulide have been isolated (Ma L., et al., 2013). The lanosterol and a derivative thereof have been found to have physiological activities including anticancer, anti-inflammatory, and anti-allergic activities.

The present inventors have completed the disclosure relating to a pharmaceutical formulation for enhancing the physiological activity of lanosterol and a derivative thereof.

### Disclosure of Invention

### Technical Problem

One aspect is to provide a pharmaceutical formulation including: a compound represented by Formula 1 or a pharmaceutically acceptable salt thereof; and a cyclodextrin derivative represented by Formula 2.

[Formula 1]

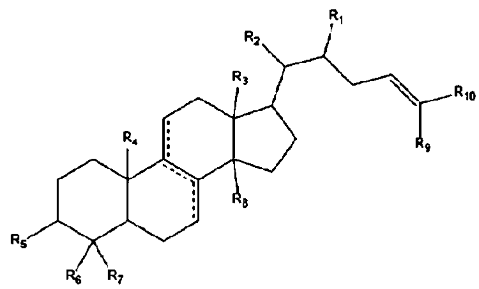

[Formula 2]

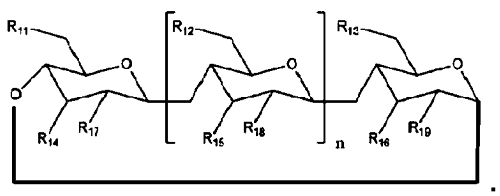

In Formula 1, $R_1$ to $R_{10}$ may independently be H, substituted or unsubstituted $C_1$-$C_4$ alkyl, substituted or unsubstituted $C_2$-$C_4$ alkenyl, substituted or unsubstituted $C_2$-$C_4$ alkynyl, substituted or unsubstituted $C_1$-$C_4$ alkoxy, halogen, hydroxy, aldehyde, carboxy, amino, nitro, or cyano, and

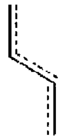

in Formula 1 may be

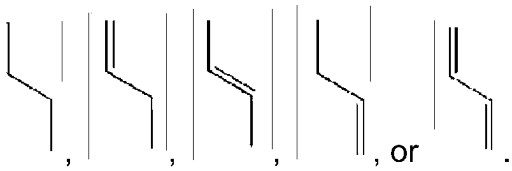

, , , , or .

In Formula 2, n may be 4, 5, or 6, and $R_{11}$, $R_{12}$, $R_{13}$, $R_{14}$, $R_{15}$, $R_{16}$, $R_{17}$, $R_{18}$, and $R_{19}$ may independently be -OH, substituted or unsubstituted $C_1$-$C_4$ alkoxy, or substituted or unsubstituted $C_1$-$C_4$ alkylthio.

Another aspect is to provide a method of preparing the formulation.

Solution to Problem

One aspect provides a pharmaceutical formulation including: a compound represented by Formula 1 or a pharmaceutically acceptable salt thereof; and a cyclodextrin derivative represented by Formula 2.

[Formula 1]

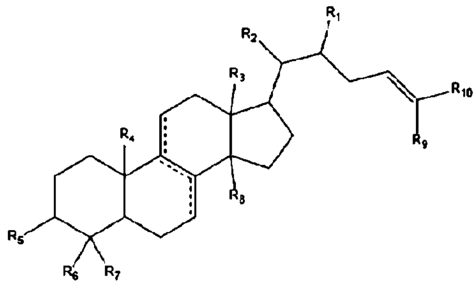

[Formula 2]

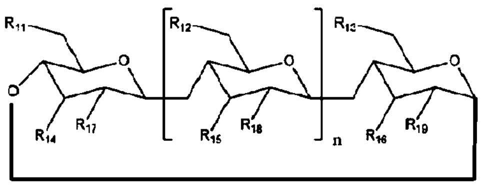

.

In Formula 1, $R_1$ to $R_{10}$ may independently be H, substituted or unsubstituted $C_1$-$C_4$ alkyl, substituted or unsubstituted $C_2$-$C_4$ alkenyl, substituted or unsubstituted $C_2$-$C_4$ alkynyl, substituted or unsubstituted $C_1$-$C_4$ alkoxy, halogen, hydroxy, aldehyde, carboxy, amino, nitro, or cyano, and

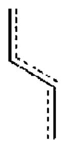

in Formula 1 may be

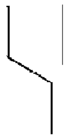, 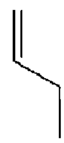, 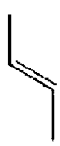, 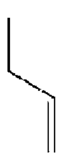, or 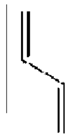.

In Formula 2, n may be 4, 5 or 6, and $R_{11}$, $R_{12}$, $R_{13}$, $R_{14}$, $R_{15}$, $R_{16}$, $R_{17}$, $R_{18}$, and $R_{19}$ may independently be -OH, substituted or unsubstituted $C_1$-$C_4$ alkoxy, or substituted or unsubstituted $C_1$-$C_4$ alkylthio.

As used in the present specification, the term 'comprising' refers to 'including', and unless otherwise implied by the context, it is intended, for example, not to exclude the presence of additional ingredients, except for the case where the ingredients together add up to 100%.

In an embodiment, the compound represented by Formula 1 may refer to lanosterol and a derivative thereof.

In the present specification, the term "lanosterol and a derivative thereof" may include at least one selected from the group consisting of lanosterol, inotodiol, trametenolic acid, ganodermadiol, and ganodermatriol.

The IUPAC name of "inotodiol" is (3S,5R,10S,13R,14R,17R)-17-[(2S,3R)-3-hydroxy-6- methylhept-5-en-2-yl]-4,4,10,13,14-pentamethyl-2,3,5,6,7,11,12,15,16,17-decahydro-1H-cyclopenta[a]phenanthren-3-ol. The inotodiol is a main bioactive substance of Chaga mushrooms (*Inonotus obliquus*), and may be chemically synthesized according to methods in the art. It may be manufactured as a pharmaceutically acceptable salt, or separated and purified from Chaga mushroom extracts. The inotodiol compound may exist in the form of a pharmaceutically acceptable salt.

In the present specification, the term "pharmaceutically acceptable salt" includes both an addition salt of acid or base and a stereochemical isomer thereof, and may be, for example, an addition salt of organic acid or inorganic acid. The salt may include any salt that maintains the activity of a parent compound in a target to be administered without causing undesirable effects, and is not particularly limited.

The salt may include an inorganic salt and an organic salt, and may be, for example, acetic acid, nitric acid, aspartic acid, sulfonic acid, sulfuric acid, maleic acid, glutamic acid, formic acid, succinic acid, phosphoric acid, phthalic acid, tannic acid, tartaric acid, hydrobromic acid, propionic acid, benzenesulfonic acid, benzoic acid, stearic acid, lactic acid, bicarbonic acid, bisulfuric acid, bitartaric acid, oxalic acid, butylic acid, calcium edetate, carbonic acid, chlorobenzoic acid, citric acid, edetic acid, toluenesulfonic acid, fumaric acid, gluceptic acid, ecylinic acid, pamoic acid, gluconic acid, methylnitric acid, malonic acid, hydrochloric acid, hydroiodoic acid, hydroxynaphtholic acid, isethionic acid, lactobionic acid, mandelic acid, mucic acid, naphthalenesulfonic acid, muconic acid, p-nitromethanesulfonic acid, hexamic acid, pantothenic acid, monohydrogen phosphoric acid, dihydrogen phosphoric acid, salicylic acid, sulfamine acid, sulfanilic acid, or methanesulfonic acid.

In addition, the salt form may include: salts of alkali and alkaline earth metals such as an ammonium salt, a lithium salt, a sodium salt, a potassium salt, a magnesium salt, and a calcium salt; for example, salts with organic bases such as benzathine, N-methyl-D-glucamine, and hydrabamine salts; and salts with amino acids such as arginine and lysine. In addition, the salt form may be converted into a free form by treatment with a suitable base or acid.

In the present specification, the term "inclusion" refers to a stoichiometric molecular phenomenon in which a guest molecule interacts with a cavity of a cyclodextrin molecule and is subsequently trapped.

In the present specification, the term "cyclodextrin (CD)" refers to a cyclic compound linked via $\alpha$-(1$\rightarrow$4)-glycosidic bonds with glucose as a basic unit. The CD is a ring-shaped sugar particle with a cavity, and may be classified into alpha ($\alpha$), beta ($\beta$), and gamma ($\gamma$) types depending on the structure. The internal pore according to the structure of the CD may be 5.3 Å for $\alpha$-CD, 6.5 Å for $\beta$-CD, and 8.3 Å for $\gamma$-CD. The CD exhibits hydrophilicity due to the presence of hydroxyl groups on the outside of the ring, while the inside is hydrophobic. Substances having a molecular structure suitable for the internal structure of the CD may be enclosed in the hydrophobic internal cavity of the CD to form an inclusion compound. In an embodiment, the inotodiol may be enclosed in the internal cavity of the CD.

In an embodiment, the CD derivative represented by Formula 2 may be $\gamma$-CD represented by Formula 3.

[Formula 3]

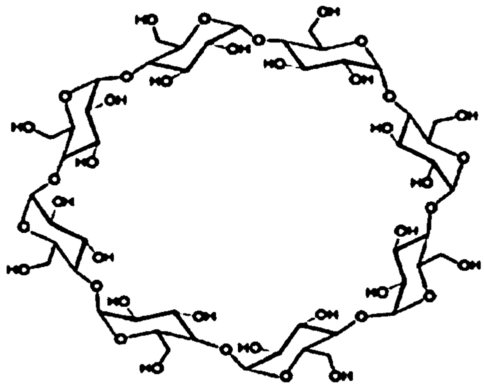

In an embodiment, the pharmaceutical formulation may be a mixture of: lanosterol and a derivative or pharmaceutically acceptable salt thereof; and CD, in a molar ratio of 1:1 to 10. Specifically, the formulation may be a mixture of: lanosterol and a derivative thereof; and CD, in a molar ratio of 1:1 to 10, 1:1 to 9, 1:1 to 8, 1:1 to 7, 1:1 to 6, 1:1 to 5, 1:1 to 4, 1:1 to 3, or 1:1 to 2. For example, inotodiol and CD may be mixed in a molar ratio of 1:3 to form an inclusion complex in which inotodiol is enclosed in the cavity of CD.

Furthermore, the pharmaceutical formulation may enhance the water solubility of hydrophobic lanosterol or a derivative thereof by enclosing lanosterol or a derivative thereof in a CD derivative. In an embodiment, the pharmaceutical formulation may increase the bioavailability and anti-inflammatory effect of lanosterol or a derivative thereof.

Meanwhile, in an embodiment, the formulation may be a solid formulation or a liquid formulation.

The solid formulation may be prepared as any one selected from the group consisting of a tablet, a capsule, a powder, a dispersible granule, a cachet, a suppository, a sustained-release formulation, and a delayed-release formulation.

The liquid formulation may be prepared as any one selected from the group consisting of a solution, a suspension, an emulsion, an injectable formulation, a solution or spray for nasal, buccal mucosal, or sublingual administration, an aerosol formulation suitable for inhalation, a transdermal formulation, a cream, a lotion, and a formulation suitable for incorporation into a transdermal patch.

In an embodiment, the formulation may further include a pharmaceutically acceptable additive.

The additive may be at least one selected from the group consisting of a diluent, a binder, a disintegrant, and a lubricant.

The diluent may be at least one selected from the group consisting of lactose, cellulose powder, microcrystalline cellulose, silicified microcrystalline cellulose, starch, gelatinized starch, calcium carbonate, cyclodextrin, calcium sulfate, calcium silicate, magnesium carbonate, dicalcium phosphate, tricalcium phosphate, magnesium trisilicate, potassium chloride, sodium chloride, calcium phosphate dibasic dihydrate, calcium phosphate tribasic, kaolin, magnesium carbonate, magnesium oxide, mannitol, maltitol, sorbitol, xylitol, lactose, dextrose, maltose, sucrose, glucose, fructose, maltodextrin, dextrate, dextrin, and a combination.

The binder may be at least one selected from the group consisting of povidone, hydroxypropyl cellulose, hydroxypropyl methylcellulose, light anhydrous silicic acid, aluminum silicate, calcium silicate, calcium hydrogen phosphate, calcium carbonate, and a combination thereof.

The disintegrant may be at least one selected from the group consisting of croscarmellose sodium (CrosCMC-Na), carboxymethyl cellulose, cross-linked polyvinylpyrrolidone (crospovidone), low-substituted hydroxypropyl cellulose (L-HPC), starch, sodium carboxymethyl starch, sodium starch glycolate, partially hydrolyzed starch, and a combination thereof.

The lubricant may be at least one selected from the group consisting of magnesium stearate, fumaric acid, stearic acid, calcium stearate, sodium stearyl fumarate, polyethylene glycol, starch, talc, highly dispersed (colloidal) silica, magnesium oxide, magnesium carbonate, glyceryl behenate, glyceryl monostearate, silicon dioxide, calcium silicate, magnesium silicate, and a combination thereof.

The formulation may contain additional excipients commonly used in the art. Such additional excipients may include a plasticizer, a film former, a colorant, an anti-tacking agent, and/or a pigment, for coating the composition of the present disclosure. Additional types of available excipients include a buffer, a flavoring agent, a sweetener, an antioxidant, and/or an absorption enhancer.

A coating substrate for coating the formulation may be at least one selected from the group consisting of: a polymer, such as polyvinyl pyrrolidone (PVP), hydroxypropyl methylcellulose (HPMC), carboxymethyl cellulose (sodium salt and calcium salt), ethyl cellulose, methyl cellulose, hydroxy ethyl cellulose, ethyl hydroxyethyl cellulose, hydroxypropyl cellulose (HPC), L-HPC, polyvinyl alcohol, acrylic acid, and salts of thereof; a vinylpyrrolidone-vinylacetate copolymer; a gelatin; a guar gum; a partially hydrolyzed starch; alginate; xanthan; and a combination thereof.

The inclusion compound including: the compound represented by Formula 1 or a pharmaceutically acceptable salt thereof; and the CD derivative represented by Formula 2 may be purified by any method known to those skilled in the art, if necessary. It should be noted that those skilled in the art are familiar with purification methods when such purification methods are practiced. For example, in multi-step synthesis that aids in reaching a specific compound, purification may be performed after all synthetic processes, after several processes, at various points during synthesis, and/or at the very end of synthesis. In some methods, one or more purification processes include techniques selected from the group consisting of silica gel column chromatography, C-18 reverse-phase column chromatography, gel permeation column chromatography, high-performance liquid chromatography (HPLC), and liquid chromatography (LC).

Another aspect provides a manufacturing method for a formulation, the method including mixing: a compound represented by Formula 1 or a pharmaceutically acceptable salt thereof; and a CD derivative represented by Formula 2.

[Formula 1]

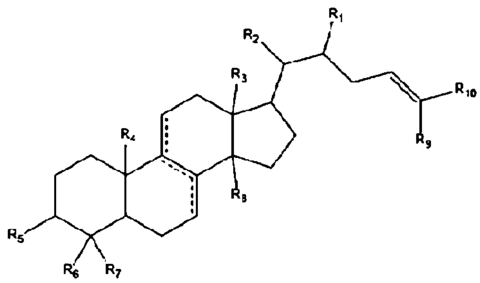

[Formula 2]

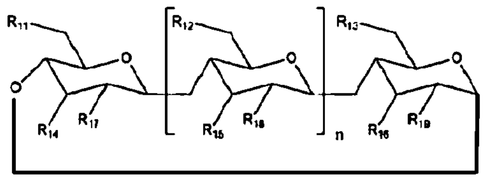

In Formula 1, $R_1$ to $R_{10}$ may independently be H, substituted or unsubstituted $C_1$-$C_4$ alkyl, substituted or unsubstituted $C_2$-$C_4$ alkenyl, substituted or unsubstituted $C_2$-$C_4$ alkynyl, substituted or unsubstituted $C_1$-$C_4$ alkoxy, halogen, hydroxy, aldehyde, carboxy, amino, nitro, or cyano, and

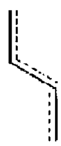

in Formula 1 may be

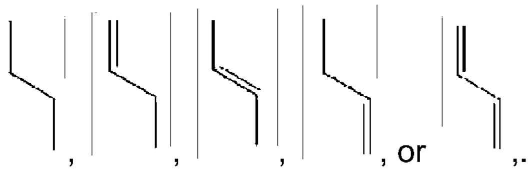

, , , , or ,.

In Formula 2, n may be 4, 5 or 6, and $R_{11}$, $R_{12}$, $R_{13}$, $R_{14}$, $R_{15}$, $R_{16}$, $R_{17}$, $R_{18}$, and $R_{19}$ may independently be -OH, substituted or unsubstituted $C_1$-$C_4$ alkoxy, or substituted or unsubstituted $C_1$-$C_4$ alkylthio.

In an embodiment, the compound represented by Formula 1 may refer to lanosterol and a derivative thereof. The lanosterol and a derivative thereof, a pharmaceutically acceptable salt thereof, and CD are as described above.

In an embodiment, the mixing may include mixing the compound represented by Formula 1 or a pharmaceutically acceptable salt thereof, and the CD represented by Formula 2, in a molar ratio of 1:1 to 10.

The manufacturing method may utilize an ultrasonic method, a highpressure homogenizer, a high- temperature treatment method, a lyophilization method, a kneading method, or a co-evaporation method.

In an embodiment, the manufacturing method may further include adding a surfactant to mixture. The surfactant may include Tween 20, Tween 80, or sucrose ester, but is not limited thereto. The surfactant may be added in a concentration of 0.1 to 5 %.

## Advantageous Effects of Invention

According to the pharmaceutical formulation of one aspect, the water solubility, stability in solution, and bio absorbability are enhanced, leading to increased bioavailability and enhanced anti-inflammatory effects.

## Brief Description of Drawings

FIG. 1 is a schematic diagram explaining a method of producing a complex in which inotodiol is enclosed in cyclodextrin.

FIG. 2 is a diagram illustrating a complex in which inotodiol is enclosed in cyclodextrin.

FIG. 3 is a graph confirming formation of an inclusion complex of cyclodextrin and inotodiol through differential scanning calorimetry (DSC) analysis:

FIG. 3A is a graph showing results of DSC analysis after inotodiol is enclosed in beta-cyclodextrin, and FIG. 3B is a graph showing results of DSC analysis after inotodiol is enclosed in gamma-cyclodextrin.

FIG. 4 is a graph showing formation of an inclusion complex according to a mixing ratio of inotodiol and cyclodextrin.

FIG. 5 is a graph showing a decomposition rate of an inclusion complex of inotodiol and cyclodextrin over time.

FIG. 6 shows results of evaluating anti-inflammatory efficacy against LPS by using a mouse-derived macrophage cell line (Raw264.7):

FIG. 6A is an image showing expression levels of IL-6, IL-1$\beta$, and TNF-$\alpha$ by unenclosed inotodiol and an inclusion complex of inotodiol and cyclodextrin, FIG. 6B is a graph showing relative expression levels of IL-6, FIG. 6C is a graph showing relative expression levels of IL-1$\beta$, and FIG. 6D is a graph showing relative expression levels of TNF-$\alpha$.

FIG. 7 is a graph showing results of measuring $EC_{50}$ of unenclosed inotodiol and an inotodiol/$\gamma$-cyclodextrin (CD) inclusion complex in a human mast cell line (LUVA cell).

FIG. 8 shows anti-inflammatory effect of an inotodiol/CD inclusion complex in a systemic mouse model of sepsis:

FIG. 8A is a schematic diagram showing an experimental process for confirming anti-inflammatory effect in a systemic mouse model of sepsis, FIG. 8B is a graph showing results of measuring a body temperature after inducing inflammation in a mouse model of sepsis and treating the mouse model with an inclusion complex of inotodiol and CD, FIG. 8C is a graph showing results of confirming expression levels of pro-inflammatory cytokine IL-1$\beta$ after inducing inflammation in a mouse model of sepsis and treating the mouse model with an inclusion complex of inotodiol and CD, FIG. 8D is a graph showing results of confirming expression levels of pro-inflammatory cytokine TNF-$\alpha$ after inducing inflammation in a mouse model of sepsis and treating the mouse model with an inclusion complex of inotodiol and CD (naive (2 mice), sham (3 mice), Dexa 4 mpk in water (2 mice), G1: inotodiol 0.25 (ino -$\gamma$CD 0.25 mpk in saline) (4 mice), G2: inotodiol 0.5 (ino -$\gamma$CD 0.5 mpk in saline) (4 mice), G3: inotodiol 1 (ino 1 mpk in oil) (4 mice), G4: inotodiol 4 (ino 4 mpk in oil) (4 mice)).

FIG. 9 shows results of comparing dosage-dependent *in vivo* bioavailability of unenclosed inotodiol and an inclusion complex of inotodiol and CD:

FIG. 9A is a graph showing results of comparing inotodiol concentrations in serum after oral administration of unenclosed inotodiol (0.5 mpk) and an inotodiol/CD inclusion complex (0.5 mpk) to mice, FIG. 9B is a graph showing results of comparing inotodiol concentrations in serum after oral administration of unenclosed inotodiol (1 mpk) and an inotodiol/CD inclusion complex (1 mpk) to mice, and FIG. 9C is a graph showing results of comparing inotodiol concentrations in serum after oral administration of unenclosed inotodiol (4 mpk) and an inotodiol/CD inclusion complex (4) to mice.

## Mode for the Invention

Hereinafter, the following examples provide a more detailed description. However, these examples are for illustrative purposes only, and the scope of the present disclosure is not intended to be limited by these examples.

## Example 1. Manufacture of inclusion complex of inotodiol and cyclodextrin

1.1 **Manufacture of inclusion complex of inotodiol and γ-cyclodextrin**

A complex in which inotodiol is included in γ-cyclodextrin was manufactured using methods shown in FIGS. 1 and 2.

Specifically, first, γ-cyclodextrin was dissolved in 9 ml of DDW in proportion to the molar ratio with inotodiol (1:1 to 1:10). Then, during ultrasound treatment (first use: microtip, Amp 30 %, 10 minutes; second use: flat tip, Amp 50 %, 5 minutes), 10 mg of inotodiol dissolved in 1 ml of EtOH was slowly injected thereto at 15-second intervals. Next, the solution was incubated in a suspension state at 25 °C for 24 hours and dried using a freeze dryer (-45 °C, 2 to 3 days) to manufacture an inclusion complex. Additionally, various types of surfactants were added as auxiliary agents at concentrations ranging from 0.1 to 5 % to the suspension, which is in solution state after the ultrasound treatment, thereby improving the stability of the suspension.

**1.2 Confirmation of formation of inclusion complex of inotodiol and cyclodextrin according to cyclodextrin types**

The formation of the inotodiol/cyclodextrin (CD) inclusion complex prepared in Example 1 was confirmed by differential scanning calorimetry (DSC) analysis.

Specifically, 2 mg of each dried sample was placed in a sealed aluminum pan, and the pan was heated from 25° C to 310 °C at a rate of 10° C/min under a nitrogen atmosphere. An empty pan was used as a reference. The graph confirming the formation of the inotodiol/CD inclusion complex by DSC analysis is shown in FIG. 3.

FIG. 3A shows the graph obtained from DSC analysis after enclosing inotodiol in beta-cyclodextrin (β-CD), and FIG. 3B shows the graph obtained from DSC analysis after enclosing inotodiol in gamma-cyclodextrin (γ-CD).

As shown in FIGS. 3A and 3B, the melting peak (at 192 °C) of inotodiol crystals (lipid crystals) was detected in the inotodiol/β-CD inclusion complex, but was not detected in the inotodiol/γ-CD inclusion complex.

This indicates that, while cholesterol having a structure similar to inotodiol forms an inclusion complex with β-CD, inotodiol, which occupies a larger volume overall than cholesterol due to two methyl groups on the 4th carbon of the first ring and one methyl group at the 14th carbon between the third ring and the fourth ring, forms an inclusion complex only with γ-CD that has a large internal hydrophobic cavity. That is, the results above indicate that inotodiol forms an inclusion complex only with γ-CD having a large internal hydrophobic cavity.

**1.3 Confirmation of inclusion complex formation according to mixing ratio of inotodiol and CD**

The formation of inotodiol/CD inclusion complex according to the mixing ratio of inotodiol and CD was confirmed. The results are shown in Table 1 and FIG. 4.

Table 1 shows the results indicating whether the inotodiol/CD inclusion complex was formed when inotodiol and CD were mixed in a molar ratio of 1:1 to 1:5, and FIG. 4 shows a graph showing the formation of inclusion complex according to the mixing ratio (molar ratio) of inotodiol and CD.

As shown in Table 1 and FIG. 4, it was confirmed that, when inotodiol and γ-CD were mixed in a molar ratio of 1:3 to 1:5, inotodiol was completely enclosed in γ-CD.

[Table 1]

| | **Inotodiol (mg)** | **mJ** | **mJ/mg** | **Yield of complexation (%)** |
|---|---|---|---|---|
| **PM 1:5** | 0.13 | 7.91 | 61.89 | 0 |
| **1:1** | 0.51 | 26:30 | 51.68 | 16.51 |
| **1:2** | 0.29 | 12.15 | 41.67 | 32.67 |
| **1:3** | 0.20 | | | >95 |
| **1:5** | 0.13 | | | >95 |

**Experimental Example 1. Confirmation of decomposition rate of inotodiol/CD inclusion complex**

To confirm the release efficiency of inotodiol from the inotodiol/CD inclusion complex, the decomposition rate of the inotodiol/CD inclusion complex manufactured in Example 1 was confirmed.

Specifically, each of γ-CD and the inotodiol/CD inclusion complex of Example 1 was dissolved in PBS (pH 7.5) until the concentration reached 0.1 mg/ml. Afterwards, by treatment with 0.25 U/ml of porcine pancreatic α-amylase (PPA), which has the same drug-active site as the human and mouse pancreatic α-amylase and has high similarity of 87.1 % and

85.5 % to the human and mouse pancreatic α-amylase, respectively, the reaction was allowed at 37 °C and 100 rpm for 4 hours. The hydrolysis degree was measured by measuring maltose, which is a reducing sugar as a reaction product of α-amylase, by the copper bicinchoninate (CBC) method, and the results are shown in Table 2 and FIG. 5.

Table 2 shows the numerical results of the time-dependent decomposition rate of the inotodiol/CD inclusion complex, and FIG. 5 shows a graph of the time-dependent decomposition rate of the inotodiol/CD inclusion complex.

[Table 2]

| Sample | 0 min | 10 min | 30 min | 60 min | 90 min | 120 min | 150 min | 180 min | 210 min | 240 min |
|---|---|---|---|---|---|---|---|---|---|---|
| γ-CD (PPA 0.25 U) | 3.45 ±0.1 0 | 7.10 ±0.0 2 | 15.0 4 ±0.0 | 24.2 2 ±0.2 | 33.0 7 ±0.4 | 34.2 3 ±0.62 | 34.8 5 ±0.50 | 35.8 1 ±0.54 | 37.5 6 ±0.62 | 41.3 4 ±2. 41 |
| | | | 3 | 5 | 6 | | | | | |
| I no/γ-CD (PPA 0.25 U) | 4.09 ±0.2 2 | 7.69 ±0.3 5 | 13.5 7 ±0.3 8 | 25.4 6 ±0.1 7 | 30.2 0 ±0.6 6 | 33.5 7 ±0.96 | 36.8 1 ±0.04 | 37.3 1 ±0.12 | 38.7 6 ±0.58 | 45.37 ±0. 04 |

As shown in Table 2 and FIG. 5, the decomposition of the inclusion complex by PPA showed a relatively similar degradation rate to γ-CD, and both showed rapid decomposition rates from the initial stage up to 90 minutes. This indicates that the ring-opening reaction of γ-CD occurred early, leading to rapid release of inotodiol. In addition, the inotodiol/CD inclusion complex exhibited a high decomposition rate by PPA treatment, wherein the PPA has the same drug-active site as the human and mouse pancreatic α-amylase and has high similarity of 87.1 % and 85.5 % to the human and mouse pancreatic α-amylase, respectively. This indicates that the inotodiol/CD inclusion complex was applicable to humans and mice.

**Experimental example 2. Comparison of stability in solution of unenclosed inotodiol and inotodiol/CD inclusion complex**

The stability in solution of the inotodiol/CD inclusion complex manufactured in Example 1 was compared with the stability in solution of unenclosed inotodiol.

Specifically, unenclosed inotodiol or the inotodiol/CD inclusion complex was mixed with a prepared auxiliary agent. The auxiliary agent was prepared at a concentration of 100 to 15,000 ppm based on the weight of inotodiol, and then treated in a sonic bath at 50 °C for 30 minutes. Afterwards, the stability was determined by visually observing whether precipitation occurred after leaving the mixture at room temperature for 24 hours.

As a result, as shown in Table 3, it was confirmed that the inotodiol/CD inclusion complex was more stable in solution than unenclosed inotodiol.

[Table 3]

| | **ppm of inotodiol in solution** | |
|---|---|---|
| | **CE9A215** | **CE9A215/CD** |
| **Tween 80 (1 %)** | 100 < | 1500 |
| **Kolliphor (5 %)** | 500 < | 15000 |

**Experimental Example 3. Comparison of physiological activities of inotodiol and inotodiol/γ-CD inclusion complex**

**3.1 Comparison of anti-inflammatory effect in mouse-derived macrophage cell line (Raw264.7)**

The anti-inflammatory effects of the inotodiol/CD inclusion complex of Example 1 and unenclosed inotodiol were compared in a mouse-derived macrophage cell line (Raw264.7).

Specifically, Raw264.7 was seeded into a 96-well plate at a density of 0.8 X $10^6$ cells/well and cultured overnight. Then, the cells were pre-treated with inotodiol and the inotodiol/CD inclusion complex each at different concentrations for 2 hours. The cells were treated with lipopolysaccharide (LPS) at a concentration of 0.1 μg/ml and cultured for 4 hours to

induce inflammation, after which RNA was extracted. Then, the mRNA expression of IL-6, IL-1β, and TNF-α was confirmed by a reverse transcription polymerase chain reaction (RT-PCR) method, and the results are shown in FIG. 6.

FIG. 6A shows an electrophoresis image showing the mRNA expression levels of IL-6, IL-1β, and TNF-α when treated with unenclosed inotodiol and the inotodiol/CD inclusion complex, and FIGS. 6B to 6D show graphs of the quantitative analysis of bands in the electrophoresis image, expressed numerically using GAPDH as a reference. FIG. 6B shows a graph of the relative mRNA expression levels of IL-6, FIG. 6C shows a graph of the relative mRNA expression levels of IL-1β, and FIG. 6D shows a graph of the relative mRNA expression levels of TNF-α.

As shown in FIGS. 6A to 6D, in the case of unenclosed inotodiol, the mRNA expression levels of IL-6 and TNF-α were significantly reduced when treated at 5 ug/ml, and in the case of the inotodiol/γ-CD inclusion complex, the mRNA expression levels of IL-6, IL-1β, and TNF-α mRNA were significantly reduced at all concentrations. This indicates that the inotodiol/γ-CD inclusion complex exhibited more effective anti-inflammatory efficacy than unenclosed inotodiol.

### 3.2 Comparison of inhibitory effects on mast cell degranulation in LUVA cells

The inhibitory effects on mast cell degranulation in the human mast cell line (LUVA cell) by unenclosed inotodiol and the inotodiol/γ-CD inclusion complex of Example 1 in the human mast cell line (LUVA cell) were compared.

Specifically, LUVA cells were seeded into a 96-well plate at a density of 1 X $10^5$ cells/well and cultured for 1 hour. Then, the cells were treated with inotodiol and the inotodiol/CD inclusion complex each at different concentrations. After the cells were treated with IgE at a concentration of 1 μg/ml and cultured overnight, the supernatant was removed. Then, 50 μl of Tyrode buffer (containing 130 mM NaCl, 5 mM KCl, 1 mM MgCl2, 1.4 mM $CaCl_2.2H_2O$, 10 mM hepes, 5.6 mM glucose, and 0.1 % BSA) was dispensed into each well. The cells were treated with inotodiol and the inotodiol/CD inclusion complex at the same concentration as before, and then, 20 μl of a complex of 2 μM A23187 and 10 μg/ml of anti-human IgE antibody was dispensed into each well and cultured for 2 hours. 50 μ of the supernatant was collected and mixed with 50 μl of 2 mM p-nitrophenyl N-acetyl-bD-glucosamine. The mixture was then cultured for 2 hours and measured for absorbance at 405 nm. 75 μl of 0.1 mM Triton X-100 was added to the cells remaining on the plate, and the resulting cells were stored at 4 °C for 40 minutes to dissolve. Then, 50 μl of the cells was collected and mixed with 50 μl of 2 mM p-nitrophenyl N-acetyl-bD-glucosamine, cultured for 1 hour, and measured for absorbance at 405 nm. The absorbance of the supernatant and the absorbance of the cell lysate were substituted into the following equation to calculate the degranulation rate, and the drug concentration ($EC_{50}$) required to reduce the degranulation rate by half was compared to the untreated group. The results are shown in FIG. 7.

$$\%\ \text{degranulation} = \frac{Tx1.5}{Tx1.5 + Lx1.5} x100$$

(T: absorbance of supernatant, L: absorbance of cell lysate)

FIG. 7 shows a graph of the results of measuring the degranulation ratio and $EC_{50}$ when the LUVA cells were treated with unenclosed inotodiol and the inotodiol/γ-CD inclusion complex.

As shown in FIG. 7, it was confirmed that the inotodiol/γ-CD inclusion complex effectively inhibited the mast cell degranulation at a relatively low concentration compared to unenclosed inotodiol.

Furthermore, when inflammatory stimulation was induced using two types, i.e., calcium ionophore and PMA, the required concentration of drug that could inhibit mass cell degranulation by at least 50 % was confirmed to be about half lower in the inotodiol/γ-CD inclusion complex than in unenclosed inotodiol.

### 3.3 Comparison of anti-inflammatory effects in systemic mouse model of sepsis

The anti-inflammatory effect of the inotodiol/CD inclusion complex of Example 1 was confirmed in a systemic mouse model of sepsis.

Specifically, the effect of the inotodiol/CD inclusion complex of Example 1 on clinical symptoms caused by macrophages activated by LPS was confirmed in a systemic mouse model of sepsis. As shown in FIG. 8A, mice were pretreated with, based on the concentration of inotodiol, 0.5 and 4 mg/kg of an inclusion complex produced by mixing inotodiol and γ-CD at 1:3 for 4 consecutive days, and then LPS (5 mg/kg) was injected intraperitoneally 1 hour after the last treatment.

Meanwhile, the body temperature of the mice was measured by the rectal temperature every 2 hours a day, starting 4 hours after the LPS-induced inflammation. In addition, proinflammatory cytokines TNF-α and IL-1β induced by the LPS-activated macrophages were measured by ELISA using mouse blood samples collected 2 hours and 6 hours, respectively, after the LPS-induced inflammation.

The anti-inflammatory effects of the inotodiol/CD inclusion complex confirmed through the experimental process

above are shown in FIGS. 8B to 8D.

FIG. 8B shows a graph of the results of measuring the body temperature after inducing inflammation in the mouse model of sepsis and treating the mouse model with the inotodiol/CD inclusion complex, FIG. 8C shows a graph of the results of measuring the expression levels of proinflammatory cytokine IL-1β after inducing inflammation in the mouse model of sepsis and treating the mouse model with the inotodiol/CD inclusion complex, and FIG. 8D shows a graph of the results of confirming the expression level of proinflammatory cytokine TNF-α after inducing inflammation in the mouse model of sepsis and treating the mouse model with the inotodiol/CD inclusion complex.

In FIG. 8, Naive represents a group administered only the same amount of vehicle solution (2 mice), Sham represents a group treated only with LPS (3 mice), and Dexa represents the group treated with 4.0 mg/kg (mpk) of dexamethasone as a positive control group (2 mice). G1 represents a group treated with 0.25 mg/kg (mpk) of inotodial/γCD inclusion complex dissolved in saline (4 mice), G2 represents a group treated with 0.5 mg/kg (mpk) of inotodiol/γCD inclusion complex dissolved in saline (4 mice), G3 represents a group treated with 1 mg/kg (mpk) of inotodiol dissolved in oil (4 mice), and G4 represents a group treated with 4 mg/kg (mpk) of inotodiol dissolved in oil (4 mice).

Data are expressed as mean ± SD of mice in each group. The statistical significance was determined by Bonferroni post-hoc test following one-way ANOVA, and was indicated as follows compared to the treatment only with LPS: $p<0.005$(*), $p<0.05$(#).

As shown in FIG. 8B, the body temperature of the mice was lowest in the Sham group treated only with LPS, and highest in the Naive group administered with an equal volume of vehicle solution. The Dexa group and G1 to G4 groups showed the rectal temperature distribution between 35 and 36 °C.

This indicates that, compared to the Naive group having normal body temperature, the Sham group showed a significant decrease in the body temperature due to anaphylaxis. However, administration of inotodiol reduced the anaphylaxis response, thereby preventing or recovering hypothermia.

In addition, as shown in FIGS. 8C and 8D, it was confirmed that the expression of the proinflammatory cytokines (IL-1β and TNF-α) was reduced when treated with the inotodiol/CD inclusion complex.

Specifically, regarding the expression level of IL-1β (FIG. 8C), it was confirmed that the expression level of the pro-inflammatory cytokine (IL-1β) was significantly reduced compared to the group treated with only LPS, and also that the expression level of IL-1β was reduced when treated with the inotodiol/CD inclusion complex compared to treatment with inotodiol dissolved in oil.

This indicates that the inclusion complex not only has an anti-inflammatory effect, but also has a more significant anti-inflammatory effect than unenclosed inotodiol.

**Experimental Example 4. Comparison of bioavailability of inotodiol/CD inclusion complex**

The bioavailability of the inotodiol/CD inclusion complex of Example 1 and the bioavailability of inotodiol not enclosed in CD were compared at each concentration.

Specifically, unenclosed inotodiol was dissolved in 0.9 % saline containing 1 % Tween 80, and the inotodiol/CD inclusion complex was dissolved only in 0.9 % saline. Then, 200 μl of the formulation corresponding to 0.5, 1, and 4 mg/kg (mpk) was orally administered to ICR male mice (30 g). Blood samples were collected at regular intervals (0.5, 1, 2, 3, and 6h) after oral administration. The results of investigating whether the bioavailability was improved by measuring the concentration of inotodiol in plasma by LC-MS analysis after centrifuging the collected blood sample to separate the plasma are shown in FIG. 9.

FIG. 9A shows a graph of comparing the inotodiol concentration after oral administration of unenclosed inotodiol (0.5 mpk) and the inotodiol/CD inclusion complex (0.5 mpk) to mice, FIG. 9B shows a graph of comparing the inotodiol concentration after oral administration of unenclosed inotodiol (1 mpk) and the inotodiol/CD inclusion complex (1 mpk) to mice, and FIG. 9C shows a graph of comparing the inotodiol concentration after oral administration of unenclosed inotodiol (4 mpk) and the inotodiol/CD inclusion complex (4 mpk) to mice.

As shown in FIGS. 9A to 9C, the inotodiol/CD inclusion complex was found to have higher bioavailability than unenclosed inotodiol. Specifically, it was confirmed that the inotodiol concentration in the mouse serum was generally higher in the case of the inotodiol/CD inclusion complex inclusion complex (inotodiol/CD) than in the case of unenclosed inotodiol. This indicates that the bioavailability of the inotodiol/CD inclusion complex was higher than that of unenclosed inotodiol.

## Claims

**1.** A pharmaceutical formulation comprising:

a compound represented by Formula 1 or a pharmaceutically acceptable salt thereof; and

a cyclodextrin derivative represented by Formula 2:

[Formula 1]

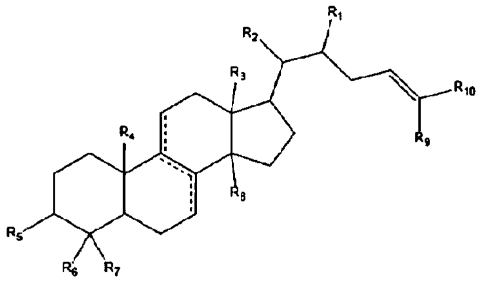

[Formula 2]

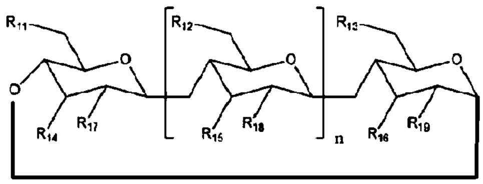

wherein, in Formula 1, $R_1$ to $R_{10}$ are independently H, substituted or unsubstituted $C_1$-$C_4$ alkyl, substituted or unsubstituted $C_2$-$C_4$ alkenyl, substituted or unsubstituted $C_2$-$C_4$ alkynyl, substituted or unsubstituted $C_1$-$C_4$ alkoxy, halogen, hydroxy, aldehyde, carboxy, amino, nitro, or cyano,

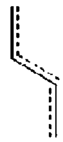

in Formula 1 is

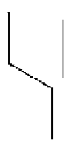, 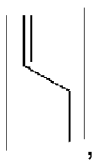, 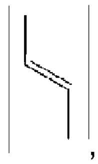, 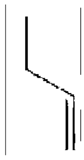, or 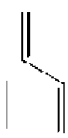,

and
in Formula 2, n is 4, 5 or 6, and $R_{11}$, $R_{12}$, $R_{13}$, $R_{14}$, $R_{15}$, $R_{16}$, $R_{17}$, $R_{18}$, and $R_{19}$ are independently -OH, substituted or unsubstituted $C_1$-$C_4$ alkoxy, or substituted or unsubstituted $C_1$-$C_4$ alkylthio.

**2.** The pharmaceutical formulation of claim 1, wherein the compound represented by Formula 1 or the pharmaceutically acceptable salt thereof is enclosed in the cyclodextrin derivative represented by Formula 2.

**3.** The pharmaceutical formulation of claim 1, wherein the compound represented by Formula 1 and the cyclodextrin derivative represented by Formula 2 are mixed in a molar ratio of 1:1 to 10.

**4.** The pharmaceutical formulation of claim 1, wherein the compound represented by Formula 1 comprises at least one selected from the group consisting of lanosterol, inotodiol, trametenolic acid, ganodermadiol, and ganodermatriol.

**5.** The pharmaceutical formulation of claim 1, wherein the cyclodextrin derivative represented by Formula 2 is gamma-cyclodextrin (γ-cyclodextrin) represented by Formula 3:

[Formula 3]

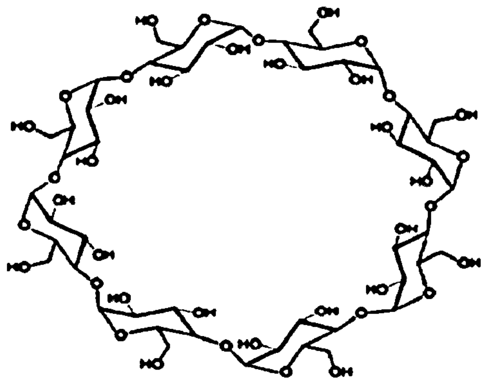

6. The pharmaceutical formulation of claim 1, wherein the formulation is a solid formulation or a liquid formulation.

7. The pharmaceutical formulation of claim 6, wherein the solid formulation is prepared as any one selected from the group consisting of a tablet, a capsule, a powder, a dispersible granule, a cachet, a suppository, a sustained-release formulation, and a delayed-release formulation.

8. The pharmaceutical formulation of claim 6, wherein the liquid formulation is prepared as any one selected from the group consisting of a solution, a suspension, an emulsion, an injectable formulation, a solution or spray for nasal, buccal mucosal or sublingual administration, an aerosol formulation suitable for inhalation, a transdermal formulation, a cream, a lotion, and a formulation suitable for incorporation into a transdermal patch.

9. The pharmaceutical formulation of claim 1 further comprising a pharmaceutically acceptable additive.

10. The pharmaceutical formulation of claim 9, wherein the additive is at least one selected from the group consisting of a diluent, a binder, a disintegrant, and a lubricant.

11. The pharmaceutical formulation of claim 10, wherein the diluent is at least one selected from the group consisting of lactose, cellulose powder, microcrystalline cellulose, silicified microcrystalline cellulose, starch, gelatinized starch, calcium carbonate, cyclodextrin, calcium sulfate, calcium silicate, magnesium carbonate, dicalcium phosphate, tricalcium phosphate, magnesium trisilicate, potassium chloride, sodium chloride, calcium phosphate dibasic dihydrate, calcium phosphate tribasic, kaolin, magnesium carbonate, magnesium oxide, mannitol, maltitol, sorbitol, xylitol, lactose, dextrose, maltose, sucrose, glucose, fructose, maltodextrin, dextrate, dextrin, and a combination.

12. The pharmaceutical formulation of claim 10, wherein the binder is at least one selected from the group consisting of povidone, hydroxypropyl cellulose, hydroxypropyl methylcellulose, light anhydrous silicic acid, aluminum silicate, calcium silicate, calcium hydrogen phosphate, calcium carbonate, and a combination thereof.

13. The pharmaceutical formulation of claim 10, wherein the disintegrant is at least one selected from the group consisting of croscarmellose sodium (CrosCMC-Na), carboxymethyl cellulose, cross-linked polyvinylpyrrolidone (crospovidone), low-substituted hydroxypropyl cellulose (L-HPC), starch, sodium carboxymethyl starch, sodium starch glycolate, partially hydrolyzed starch, and a combination thereof.

14. The pharmaceutical formulation of claim 10, wherein the lubricant is at least one selected from the group consisting of magnesium stearate, fumaric acid, stearic acid, calcium stearate, sodium stearyl fumarate, polyethylene glycol, starch, talc, highly dispersed (colloidal) silica, magnesium oxide, magnesium carbonate, glyceryl behenate, glyceryl monostearate, silicon dioxide, calcium silicate, magnesium silicate, and a combination thereof.

15. A method of preparing a formulation, the method comprising mixing a compound represented by Formula 1 or a pharmaceutically acceptable salt thereof, and a cyclodextrin derivative represented by Formula 2:

[Formula 1]

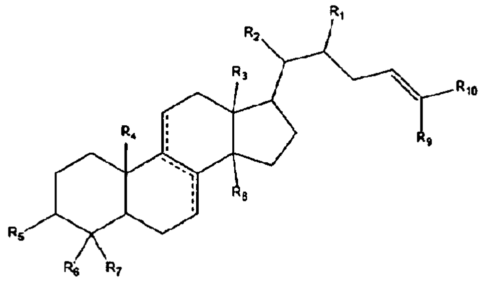

[Formula 2]

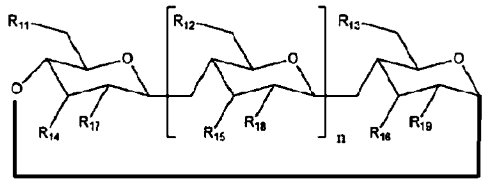

wherein, in Formula 1, $R_1$ to $R_{10}$ are independently H, substituted or unsubstituted $C_1$-$C_4$ alkyl, substituted or unsubstituted $C_2$-$C_4$ alkenyl, substituted or unsubstituted $C_2$-$C_4$ alkynyl, substituted or unsubstituted $C_1$-$C_4$ alkoxy, halogen, hydroxy, aldehyde, carboxy, amino, nitro, or cyano,

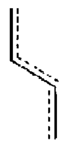

in Formula 1 is

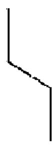, 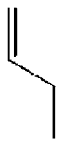, 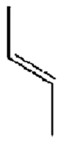, 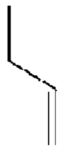, or 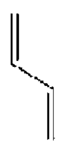,

and in Formula 2, n is 4, 5 or 6, and $R_{11}$, $R_{12}$, $R_{13}$, $R_{14}$, $R_{15}$, $R_{16}$, $R_{17}$, $R_{18}$, and $R_{19}$ are independently -OH, substituted or unsubstituted $C_1$-$C_4$ alkoxy, or substituted or unsubstituted $C_1$-$C_4$ alkylthio.

**16.** The method of preparing a formulation of claim 15, wherein the mixing comprises mixing the compound represented by Formula 1 or the pharmaceutically acceptable salt thereof, and the cyclodextrin derivative represented by Formula 2, in a molar ratio of 1:1 to 10.

**17.** The method of preparing a formulation of claim 16 further comprising adding a surfactant to mixture.

FIG. 1

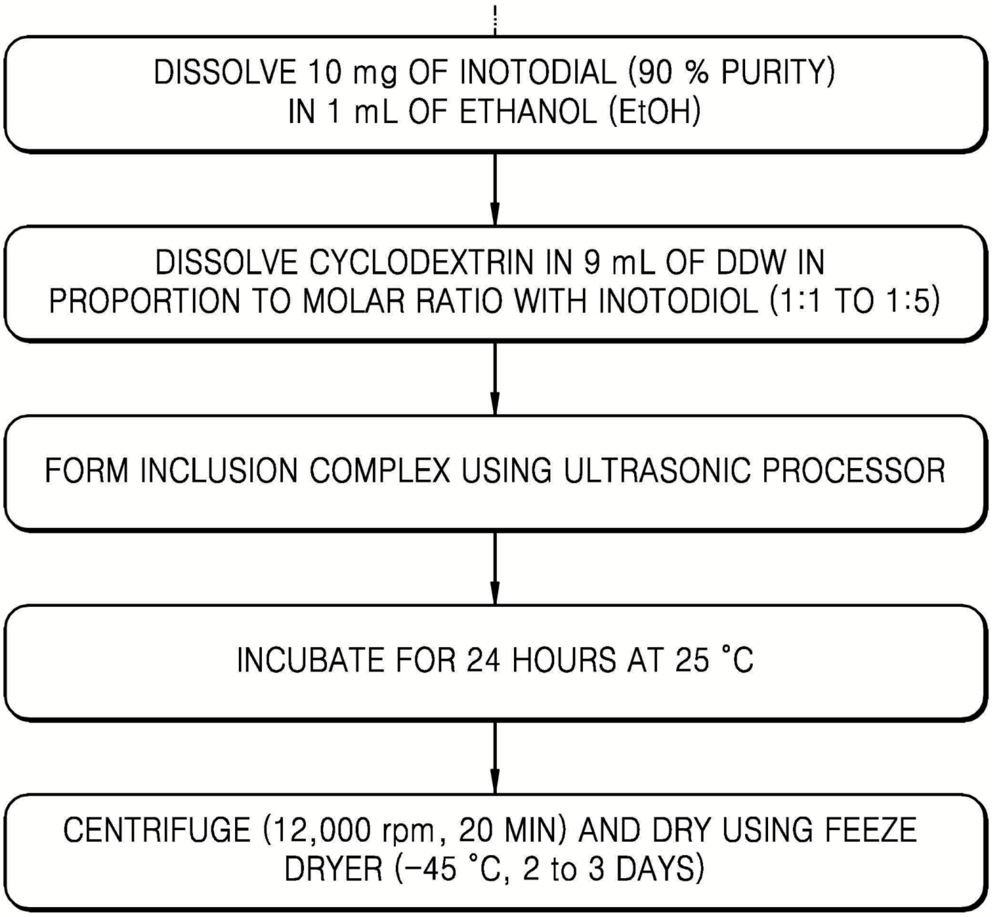
DISSOLVE 10 mg OF INOTODIAL (90 % PURITY) IN 1 mL OF ETHANOL (EtOH)
DISSOLVE CYCLODEXTRIN IN 9 mL OF DDW IN PROPORTION TO MOLAR RATIO WITH INOTODIOL (1:1 TO 1:5)
FORM INCLUSION COMPLEX USING ULTRASONIC PROCESSOR
INCUBATE FOR 24 HOURS AT 25 °C
CENTRIFUGE (12,000 rpm, 20 MIN) AND DRY USING FEEZE DRYER (−45 °C, 2 to 3 DAYS)

FIG. 2

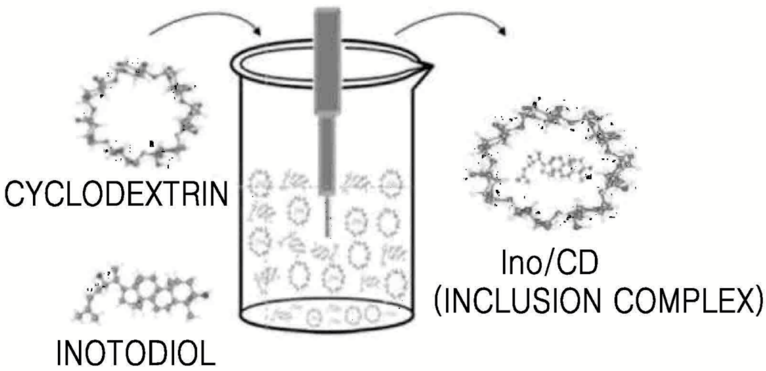

FIG. 3A

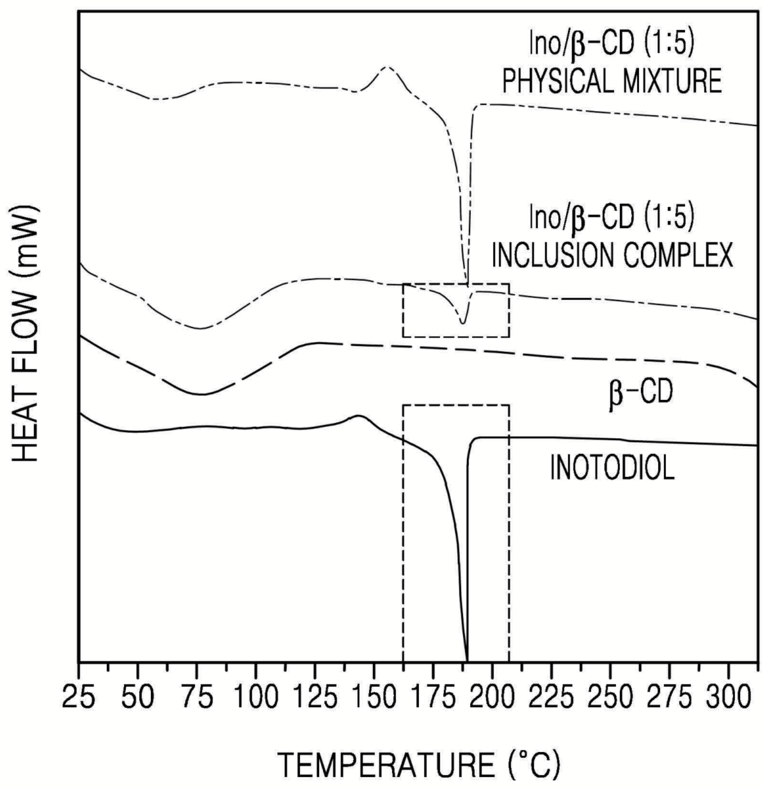
Ino/β-CD (1:5)
PHYSICAL MIXTURE
Ino/β-CD (1:5)
INCLUSION COMPLEX
β-CD
INOTODIOL
HEAT FLOW (mW)
25 50 75 100 125 150 175 200 225 250 275 300
TEMPERATURE (°C)

FIG. 3B

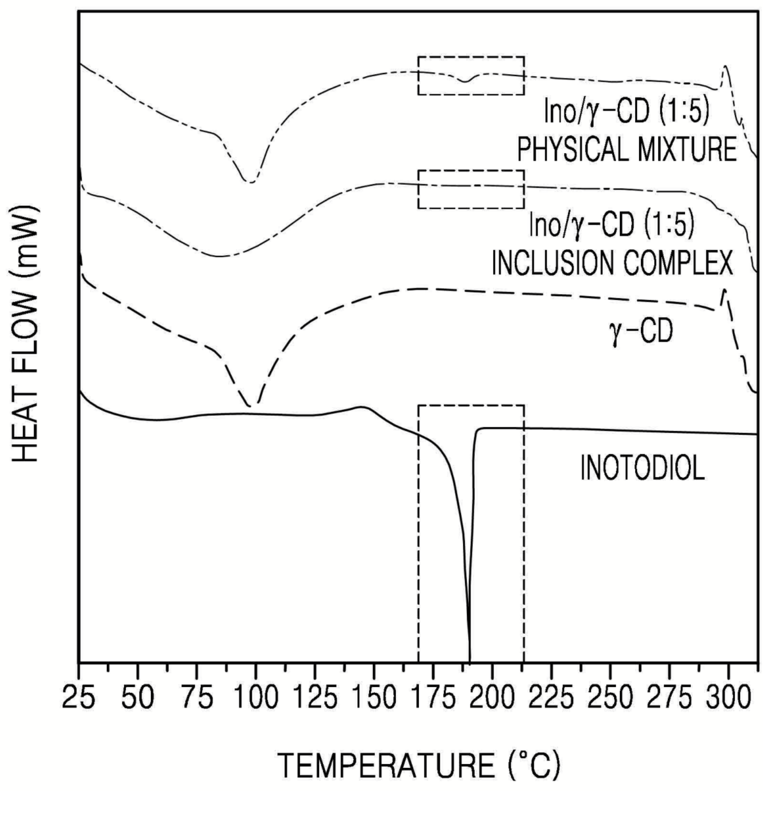

Ino/γ-CD (1:5)
PHYSICAL MIXTURE
Ino/γ-CD (1:5)
INCLUSION COMPLEX
γ-CD
INOTODIOL
HEAT FLOW (mW)
25 50 75 100 125 150 175 200 225 250 275 300
TEMPERATURE (°C)

FIG. 4

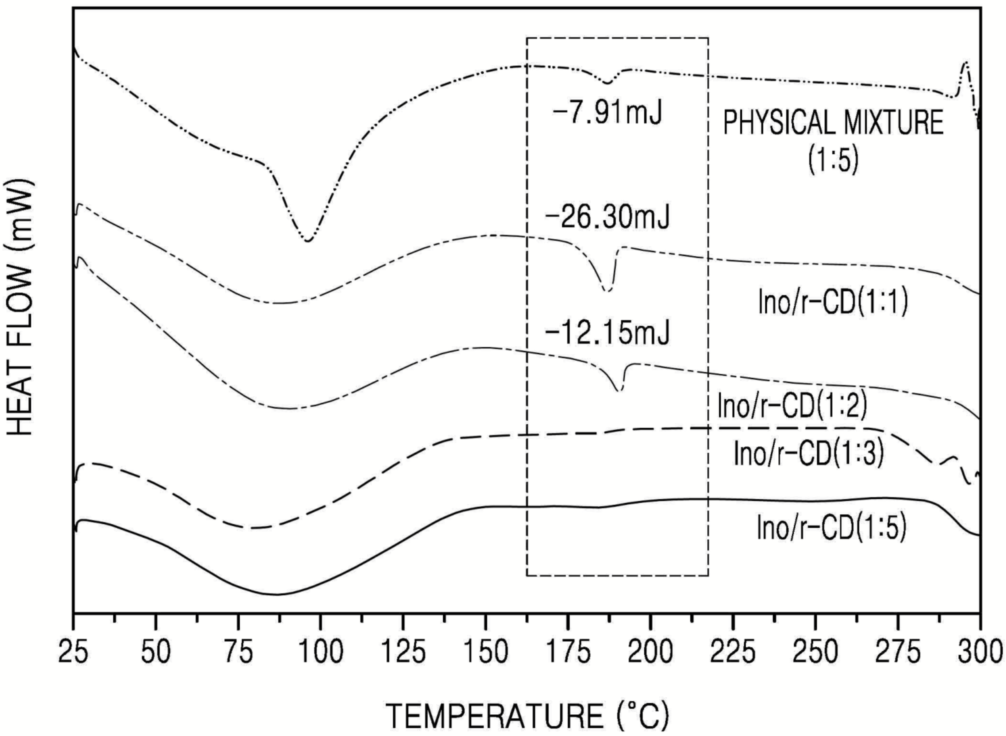
-7.91mJ
PHYSICAL MIXTURE (1:5)
-26.30mJ
Ino/r-CD(1:1)
-12.15mJ
Ino/r-CD(1:2)
Ino/r-CD(1:3)
Ino/r-CD(1:5)
HEAT FLOW (mW)
25 50 75 100 125 150 175 200 225 250 275 300
TEMPERATURE (°C)

FIG. 5

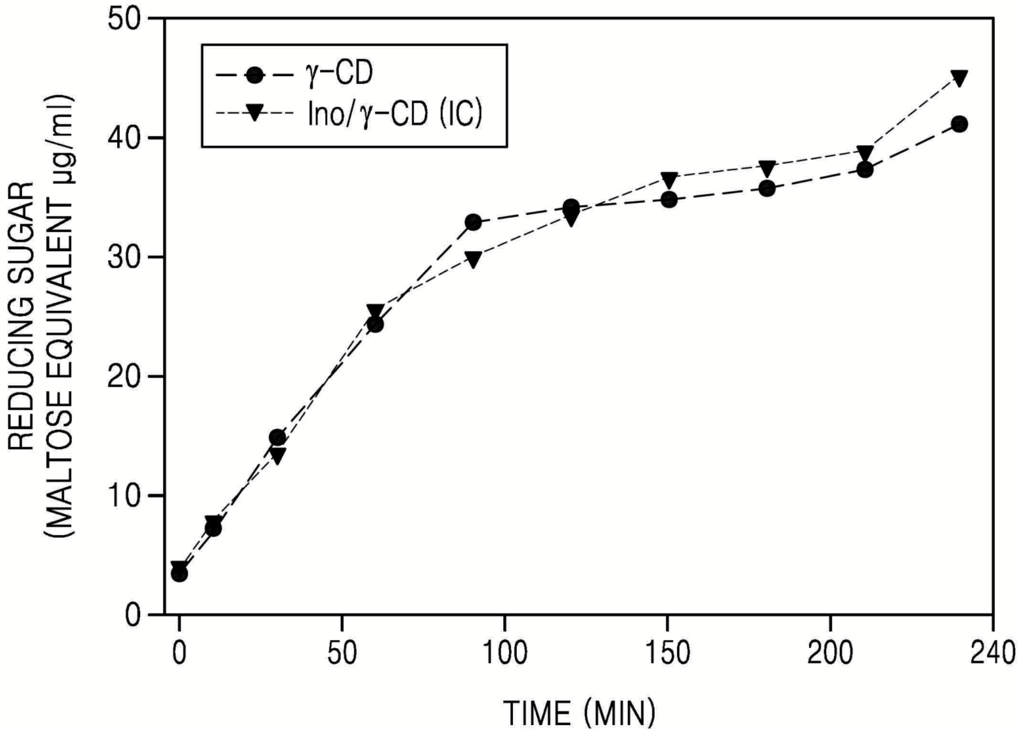
50
40
30
20
10
0
REDUCING SUGAR
(MALTOSE EQUIVALENT μg/ml)
γ-CD
Ino/γ-CD (IC)
0
50
100
150
200
240
TIME (MIN)

FIG. 6A

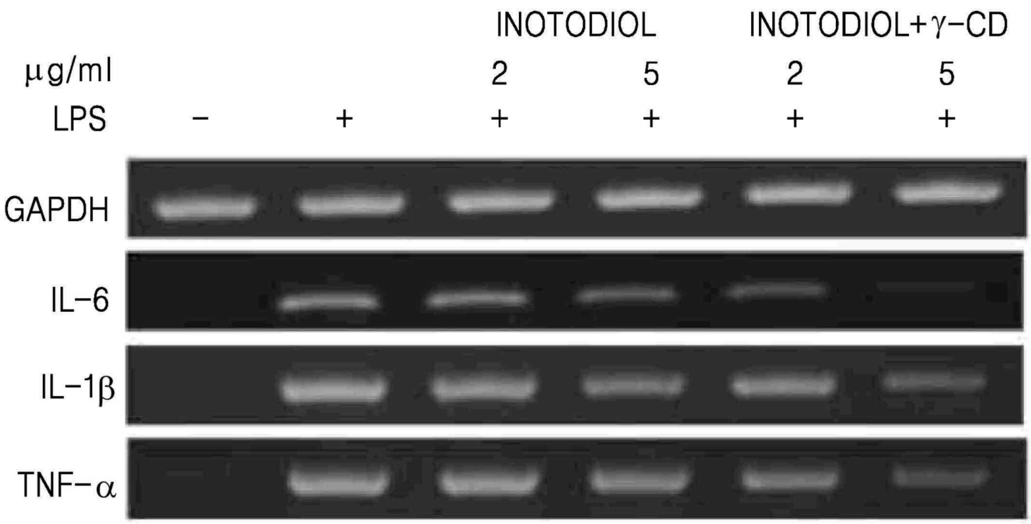

FIG. 6B

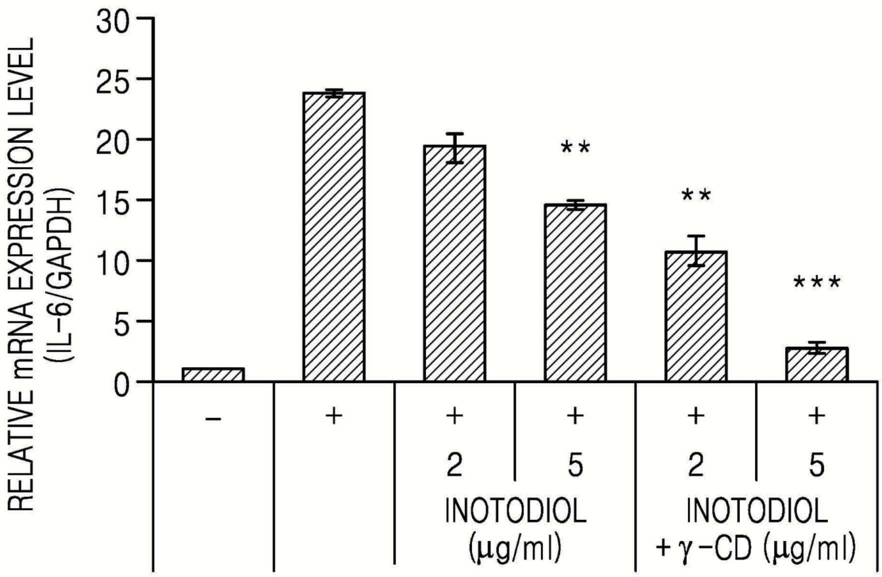


Compared with LPS(+) group: *p<0.05, **p<0.01, *p<0.001

FIG. 6C

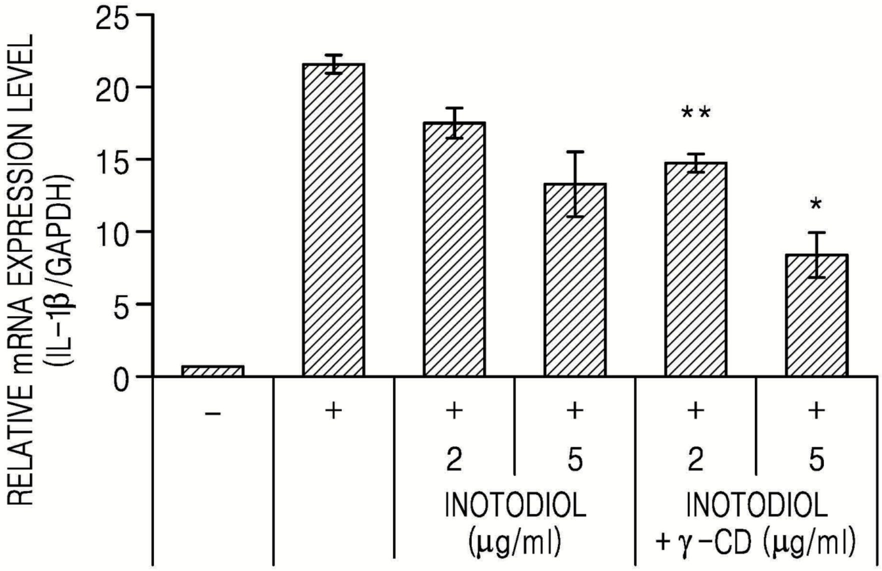
RELATIVE mRNA EXPRESSION LEVEL
(IL-1β/GAPDH)
25
20
15
10
5
0
**
*
–
+
+
+
+
+
2
5
2
5
INOTODIOL
(μg/ml)
INOTODIOL
+γ-CD (μg/ml)

FIG. 6D

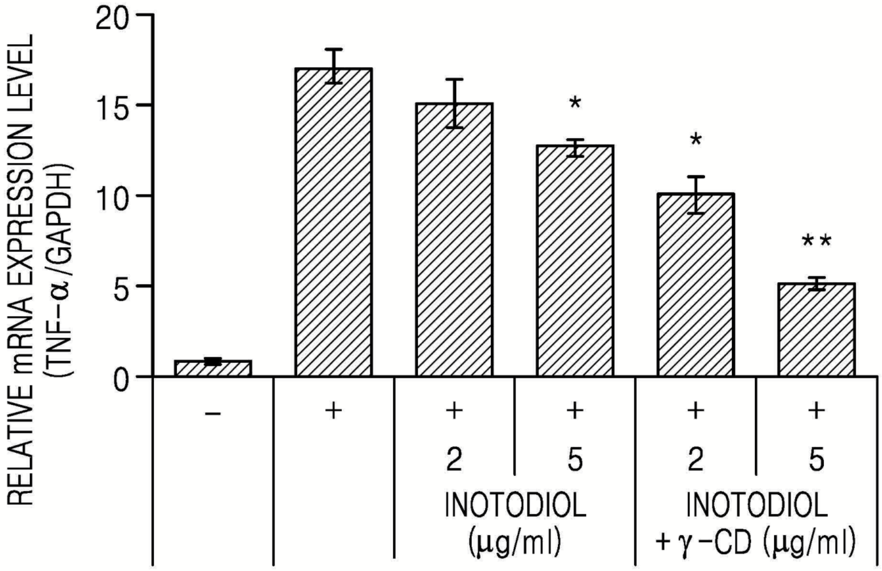
RELATIVE mRNA EXPRESSION LEVEL
(TNF-α/GAPDH)
20
15
10
5
0
*
*
**
−
+
+
+
+
+
2
5
2
5
INOTODIOL
(μg/ml)
INOTODIOL
+γ-CD (μg/ml)

FIG. 7

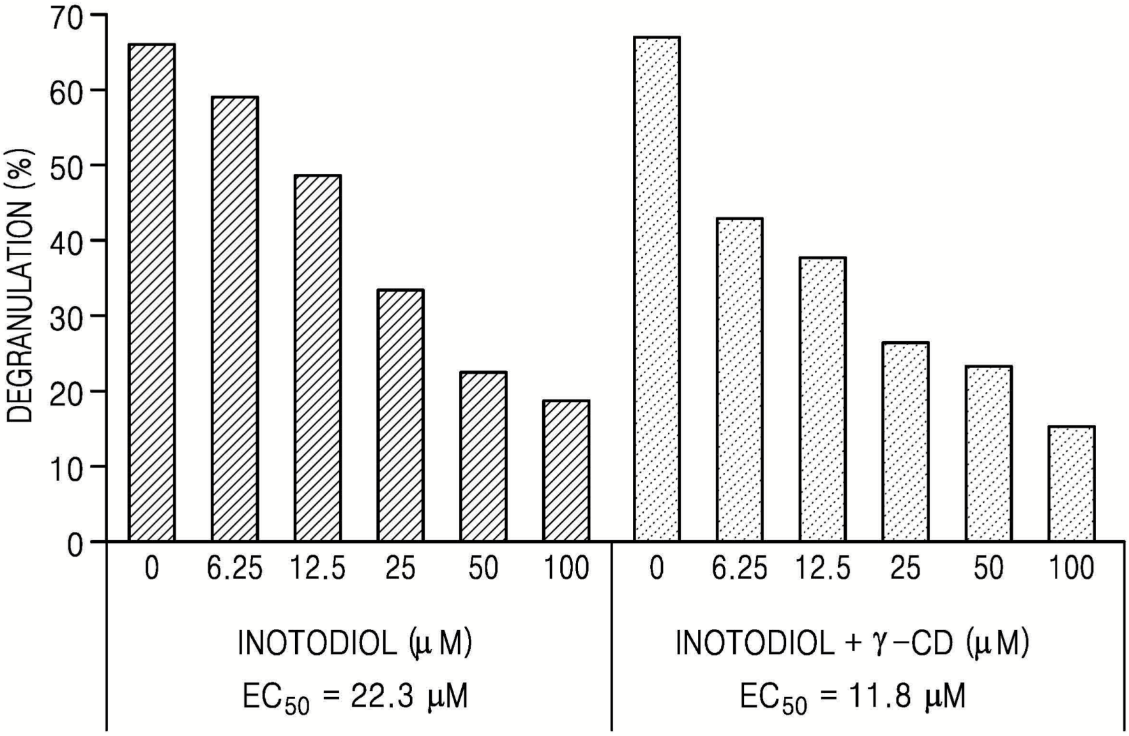
70
60
50
40
30
20
10
0
DEGRANULATION (%)
0
6.25
12.5
25
50
100
0
6.25
12.5
25
50
100
INOTODIOL (μM)
EC50 = 22.3 μM
INOTODIOL + γ-CD (μM)
EC50 = 11.8 μM

FIG. 8A

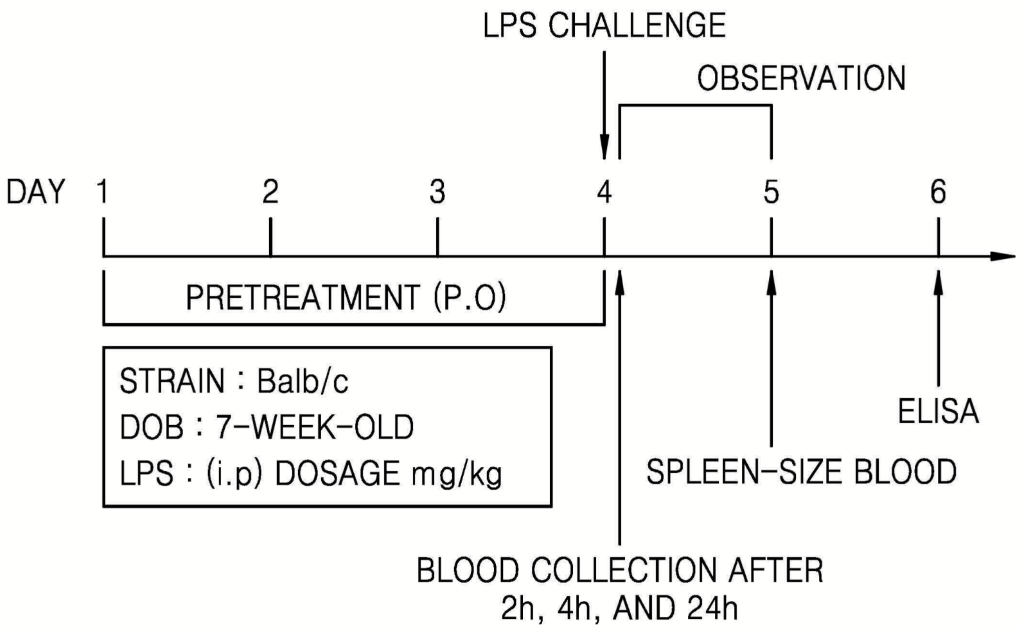
LPS CHALLENGE
OBSERVATION
DAY 1 2 3 4 5 6
PRETREATMENT (P.O)
STRAIN : Balb/c
DOB : 7-WEEK-OLD
LPS : (i.p) DOSAGE mg/kg
ELISA
SPLEEN-SIZE BLOOD
BLOOD COLLECTION AFTER
2h, 4h, AND 24h

FIG. 8B

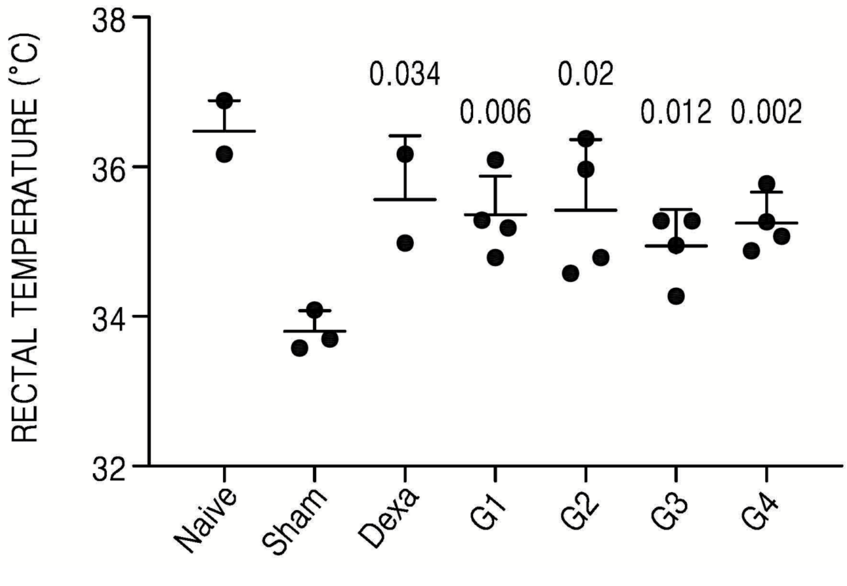
38
36
34
32
RECTAL TEMPERATURE (°C)
0.034
0.02
0.006
0.012
0.002
Naive
Sham
Dexa
G1
G2
G3
G4

FIG. 8C

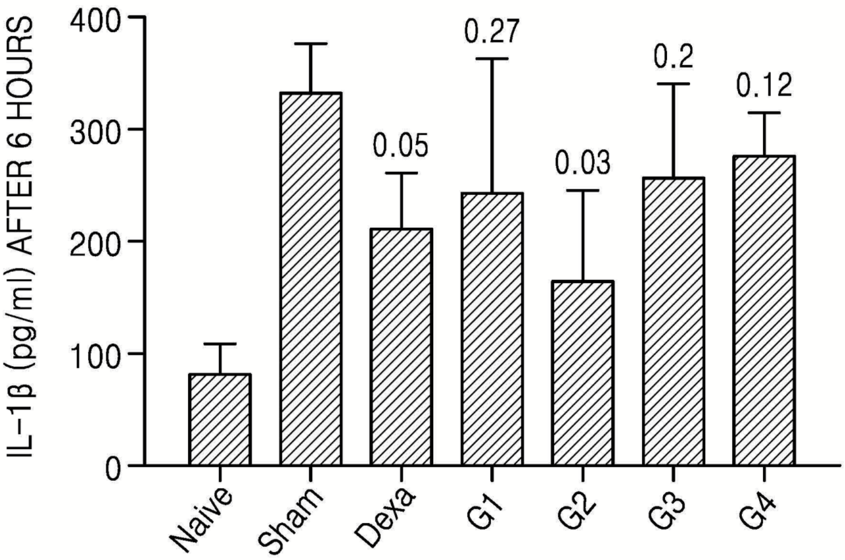
IL-1β (pg/ml) AFTER 6 HOURS
400
300
200
100
0
0.05
0.27
0.03
0.2
0.12
Naive
Sham
Dexa
G1
G2
G3
G4

FIG. 8D

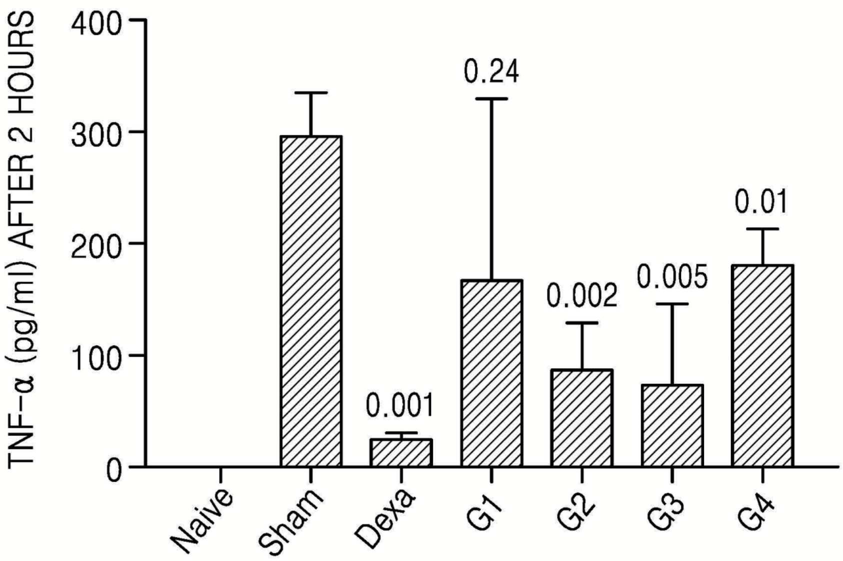
TNF-α (pg/ml) AFTER 2 HOURS
400
300
200
100
0
0.24
0.01
0.002
0.005
0.001
Naive
Sham
Dexa
G1
G2
G3
G4

FIG. 9A

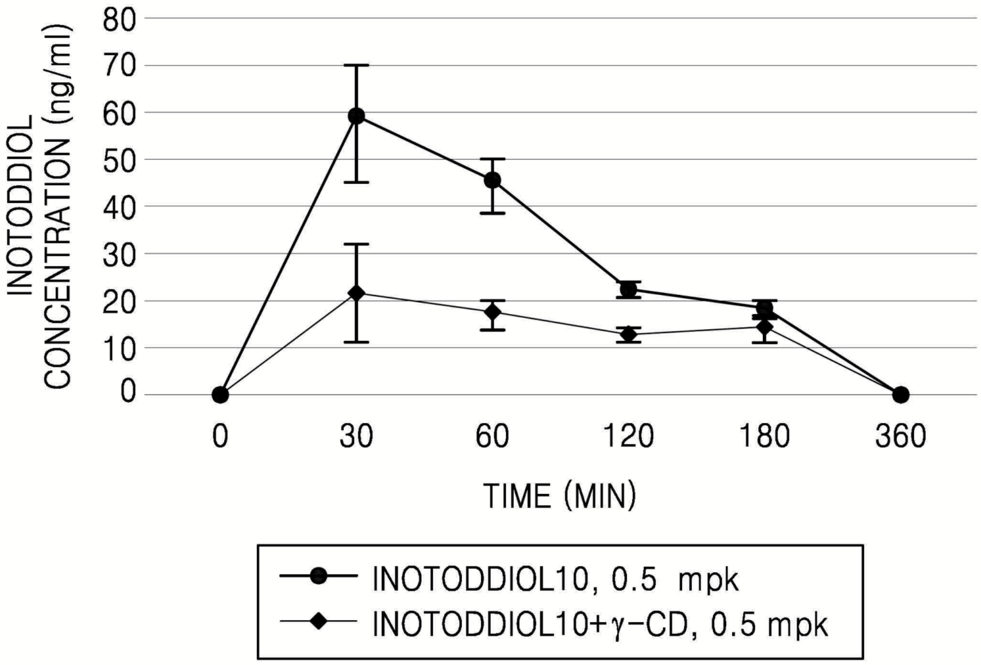
INOTODDIOL CONCENTRATION (ng/ml)
80
70
60
50
40
30
20
10
0
0
30
60
120
180
360
TIME (MIN)
INOTODDIOL10, 0.5 mpk
INOTODDIOL10+γ-CD, 0.5 mpk

FIG. 9B

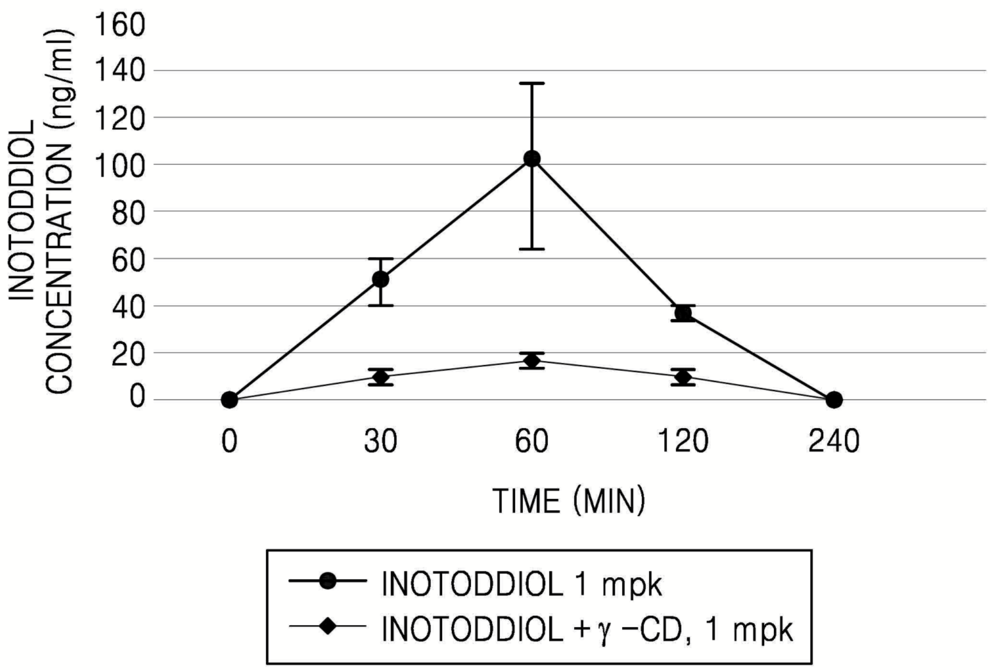

INOTODDIOL CONCENTRATION (ng/ml)
160
140
120
100
80
60
40
20
0
0
30
60
120
240
TIME (MIN)
INOTODDIOL 1 mpk
INOTODDIOL +γ -CD, 1 mpk

FIG. 9C

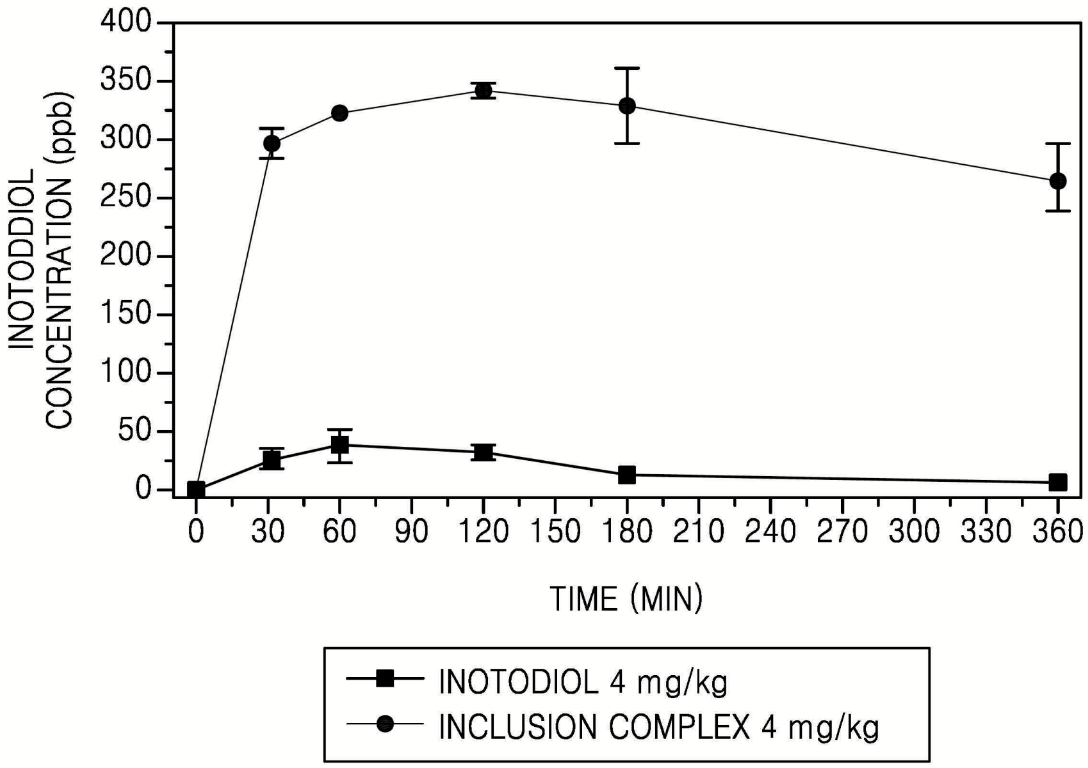
INOTODDIOL CONCENTRATION (ppb)
400
350
300
250
200
150
100
50
0
0 30 60 90 120 150 180 210 240 270 300 330 360
TIME (MIN)
INOTODIOL 4 mg/kg
INCLUSION COMPLEX 4 mg/kg

**INTERNATIONAL SEARCH REPORT**

International application No. **PCT/KR2024/005931**

**A. CLASSIFICATION OF SUBJECT MATTER**

**A61K 47/69**(2017.01)i; **A61K 31/575**(2006.01)i

According to International Patent Classification (IPC) or to both national classification and IPC

**B. FIELDS SEARCHED**

Minimum documentation searched (classification system followed by classification symbols)

A61K 47/69(2017.01); A23L 33/10(2016.01); A61K 31/575(2006.01); A61K 47/48(2006.01); C07J 9/00(2006.01); C12N 1/00(2006.01); C12Q 1/60(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus) & keywords: 포접 화합물(inclusion compound), 스테롤(sterol), 사이클로덱스트린(cyclodextrin)

**C. DOCUMENTS CONSIDERED TO BE RELEVANT**

| Category* | Citation of document, with indication, where appropriate, of the relevant passages | Relevant to claim No. |
|---|---|---|
| X | CN 102178956 A (LI, G.) 14 September 2011 (2011-09-14)<br>See claims 1, 3 and 7-9; and paragraphs [0017]-[0020]. | 1-17 |
| A | CN 101623502 A (EAST CHINA UNIVERSITY OF SCIENCE & TECHNOLOGY) 13 January 2010 (2010-01-13)<br>See entire document. | 1-17 |
| A | WO 2016-029197 A1 (ZHANG, K. et al.) 25 February 2016 (2016-02-25)<br>See entire document. | 1-17 |
| A | JP 6442808 B1 (NATIONAL UNIVERSITY CORPORATION TOKYO MEDICAL AND DENTAL UNIVERSITY et al.) 26 December 2018 (2018-12-26)<br>See entire document. | 1-17 |
| A | KR 10-2022-0018456 A (CARBOEXPERT INC.) 15 February 2022 (2022-02-15)<br>See entire document. | 1-17 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **05 August 2024** | **07 August 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.
**PCT/KR2024/005931**

**C. DOCUMENTS CONSIDERED TO BE RELEVANT**

| Category* | Citation of document, with indication, where appropriate, of the relevant passages | Relevant to claim No. |
|---|---|---|
| PX | CHOI, K.-O. et al. Enhancement of bioavailability and anti-inflammatory activity of inotodiol through complexation with γ-cyclodextrin. Journal of drug delivery science and technology. [electronic publication] 18 December 2023, vol. 91, article no. 105288, pp. 1-7. See abstract. | 1-17 |

Form PCT/ISA/210 (second sheet) (July 2022)

**INTERNATIONAL SEARCH REPORT**
**Information on patent family members**

International application No.
**PCT/KR2024/005931**

| Patent document cited in search report | Publication date (day/month/year) | Patent family member(s) | Publication date (day/month/year) |
|---|---|---|---|
| CN 102178956 A | 14 September 2011 | CN 102178956 B | 19 June 2013 |
| CN 101623502 A | 13 January 2010 | CN 101623502 B | 08 June 2011 |
| WO 2016-029197 A1 | 25 February 2016 | AU 2015-305199 A1 | 13 April 2017 |
| | | BR 112017003529 A2 | 20 August 2019 |
| | | CA 2958868 A1 | 25 February 2016 |
| | | CA 2958874 A1 | 25 February 2016 |
| | | CN 107206009 A | 26 September 2017 |
| | | CN 107206009 B | 27 March 2020 |
| | | CN 111529481 A | 14 August 2020 |
| | | CN 111529481 B | 15 September 2023 |
| | | EA 035798 B1 | 12 August 2020 |
| | | EA 201790397 A1 | 31 August 2017 |
| | | EP 3182977 A1 | 28 June 2017 |
| | | EP 3182977 B1 | 05 May 2021 |
| | | HK 1243325 A1 | 13 July 2018 |
| | | JP 2017-525769 A | 07 September 2017 |
| | | JP 6706020 B2 | 03 June 2020 |
| | | KR 10-2017-0048426 A | 08 May 2017 |
| | | MX 2017002374 A | 15 September 2017 |
| | | SG 11201701377 A | 30 March 2017 |
| | | US 10471076 B2 | 12 November 2019 |
| | | US 2017-0065617 A1 | 09 March 2017 |
| | | US 2017-0239273 A1 | 24 August 2017 |
| | | US 2019-0117675 A1 | 25 April 2019 |
| | | WO 2016-029199 A1 | 25 February 2016 |
| JP 6442808 B1 | 26 December 2018 | WO 2019-138566 A1 | 18 July 2019 |
| KR 10-2022-0018456 A | 15 February 2022 | CN 116390732 A | 04 July 2023 |
| | | EP 4193998 A1 | 14 June 2023 |
| | | EP 4193998 A4 | 20 December 2023 |
| | | JP 2023-537018 A | 30 August 2023 |
| | | KR 10-2022-0018457 A | 15 February 2022 |
| | | KR 10-2024-0011852 A | 26 January 2024 |
| | | KR 10-2567113 B1 | 18 August 2023 |
| | | KR 10-2625826 B1 | 16 January 2024 |
| | | US 2023-0302017 A1 | 28 September 2023 |
| | | WO 2022-031129 A1 | 10 February 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5362860 A

### Non-patent literature cited in the description


- **J. SZEJTLI**. *Pharmaceutical Technology*, 1991, 24-38
- **T. LOFTSSON**. *Pharmaceutical Technology*, 1999, vol. 12, 40-50
- **UEKAMA, K et al.** *CRC Critical Reviews in therapeutic Drug Carrier Systems*, 1987, vol. 3 (1), 1-40